# EUROPÄISCHE PATENTSCHRIFT

(11) **EP 2 106 249 B1**
(45) Veröffentlichungstag und Bekanntmachung des Hinweises auf die Patenterteilung: **26.08.2015**
(21) Anmeldenummer: 07857112.2
(22) Anmeldetag: 21.12.2007
(51) Int. Cl.: A61F 2/34

(54) **HÜFTGELENKPROTHESE**
HIP JOINT PROSTHESIS
PROTHÈSE ARTICULAIRE DE HANCHE

(30) Priorität: 29.12.2006 DE 102006062618
(43) Veröffentlichungstag der Anmeldung: 07.10.2009
(73) Patentinhaber: Smith & Nephew Orthopaedics AG, 6343 Rotkreuz (CH)
(72) Erfinder: GANZ, Reinhold, CH-3073 Gümlingen (CH); LEUNIG, Michael, CH-8703 Erlenbach (CH); WEHRLI, Peter, CH-5015 Erlinsbach (CH)
(74) Vertreter: Popp, Eugen
(86) Internationale Anmeldenummer: PCT/EP2007/011406
(87) Internationale Veröffentlichungsnummer: WO 2008/080595

(56) Entgegenhaltungen:
- DE-A- 2 829 676
- DE-A- 4 428 407
- DE-A- 19 843 797
- DE-A-102005 006 316
- DE-U- 9 101 766
- US-A1- 2005 060 040

## Beschreibung

Die vorliegende Erfindung betrifft eine Hüftgelenkprothese bestehend aus einer metallenen Außenschale und einem in der Außenschale verankerbaren Inlay, welches eine artikulierende bzw. Lagerfläche für einen femuralen Gelenkkopf umfaßt. Des weiteren betrifft die vorliegende Erfindung auch eine Hüftgelenkprothese mit nur einer einstückigen Gelenkschale mit artikulierender Fläche für einen femuralen Gelenkkopf.

Derartige Hüftgelenkprothesen sind allgemein bekannt. Es wird diesbezüglich nur beispielhaft auf die EP 0 346 270 A1 oder US 5 916 270 (Lipman) hingewiesen, wobei im letztgenannten Fall Maßnahmen vorgeschlagen werden, um die Gefahr einer Dislokation des Gelenkkopfes aufgrund einer Kollision (Impingement) zwischen Hüftprothesenhals und Pfannenrand zu vermeiden, zumindest zu reduzieren. Diese Maßnahmen bestehen darin, daß der sich an die artikulierende Fläche anschließende Umfangsrand von innen nach außen hin abgeschrägt ist.

Das Dokument US 2005/0060040 offenbart eine Hüftgelenksprothese, bei der eine erhöhte Mobilität des Hüftgelenkhalses in der Prothesenschale dadurch erreicht wird, dass die Ränder der Prothesenschale den natürlichen Formen des Acetabulums angeglichen sind.

Die Gefahr einer Dislokation besteht auch nach einem sogenannten "Resurfacing", d.h. nach Vornahme eines Oberflächenersatzes für die natürlichen Gleit- oder Artikulationsflächen des Acetabulums und des Hüftgelenkkopfes; denn nach einem solchen "Resurfacing" wird das Kopfdurchmesser-Schenkelhals-Verhältnis verändert, und zwar meist nachteilig hinsichtlich der Gefahr einer Dislokation.

Der vorliegenden Erfindung liegt die Aufgabe zugrunde, eine Hüftgelenkprothese, insbesondere eine Gelenkschale zur Verfügung zu stellen, bei der die vorgenannten Nachteile vermieden werden, mit der also insbesondere die Gefahr eines unzulässigen "Impingement" zwischen Schenkelhals und Pfannenrand vermieden bzw. reduziert wird, und zwar auch nach einem sogenannten "Resurfacing". Des weiteren soll durch die Erfindung eine Weichteilverletzung durch "Impingement" vermieden werden. Auch gilt es, die Gefahr einer Verletzung der Psoassehne durch Reibbewegung über den Pfannenrand hinweg zu reduzieren. Schließlich liegt der vorliegenden Erfindung auch noch die Aufgabe zugrunde, für sogenannte Flachprofilpfannen eine ebenso hohe Primärstabilität zu erreichen wie sie mit sogenannten 180°-Pfannen erhalten wird. Es gilt also, eine Pfanne mit optimiertem ROM, verbesserter Primärstabilität und hoher Variabilität bezüglich ihres Einsatzes zur Verfügung zu stellen.

Diese Aufgabe wird durch die Maßnahmen des Anspruches 1 gelöst, wobei vorteilhafte Ausführungsformen und konstruktive Details in den Unteransprüchen beschrieben sind. Dadurch, daß die Außenschale und/oder das dazugehörige Inlay, oder für den Fall, daß die Gelenkschale einstückig ausgebildet ist, diese vorzugsweise wenigstens anterior eine sich unter ihre bzw. unterhalb ihrer Äquatorialebene erstreckende Aussparung aufweist, kann sich der Schenkelhals kollisionsfrei in diese Aussparung hineinbewegen. Die Gefahr einer Kollision bzw. eines sogenannten "Impingement" besteht dann nicht mehr. Durch die erfindungsgemäß vorgesehene Aussparung wird auch die Primärstabilität der Pfanne im Acetabulum nicht beeinträchtigt. Dies gilt insbesondere auch für sogenannten Flachprofilpfannen, die sich über weniger als 180° erstrecken. Aufgrund der reduzierten Impingement-Gefahr ist natürlich in entsprechender Weise auch die Gefahr einer Dislokation vermindert bzw. vollständig vermieden.

Vorzugsweise weist die Außenschale wenigstens zwei Aussparungen auf, derart, daß sie für eine Implantation sowohl links als auch rechts geeignet ist.

Das Inlay kann in gleicher Weise ausgespart sein. Auch ist es denkbar, nur das Inlay auszusparen, und zwar ausgehend vom Umfangsrand der artikulierenden Fläche. Die vorgenannten Maßnahmen gelten auch für eine einstückige Schale aus zumindest im Bereich der artikulierenden Fläche abriebfestem Kunststoff, z.B. hochvernetztem Polyethylen, Keramik oder auch Metall. Das entsprechende Material muß natürlich humanverträglich sein.

Erfindungsgemäß befinden sich die Aussparungen in den für den Bewegungsumfang (ROM) kritischen Zonen. Dazwischen liegen vorzugsweise Segmente, welche Teil einer 180°-Pfanne sind, so daß eine hohe Primärstabilität im Acetabulum gesichert ist. Wie bereits erwähnt, ist die Anordnung der Aussparung(en)/Segmente vorzugsweise dergestalt, daß sich eine Symmetrie ergibt, wodurch die gleiche Pfanne rechts und links verwendet werden kann.

Die zwischen der oder den Aussparung(en) angeordneten Segmente können sich bis zur oder vorzugsweise über die Äquatorialebene erstrecken. Es ist jedoch auch denkbar, daß wenigstens eines dieser Segmente unterhalb der Äquatorialebene liegt.

Eine besonders vorteilhafte Ausführungsform ist dadurch gekennzeichnet, daß die Außenschale drei etwa gleichmäßig über den Umfang verteilt angeordnete Aussparungen aufweist, wobei eine davon tiefer und länger ist als die beiden anderen. Gleiches gilt für ein zugeordnetes Inlay oder eine einstückige Schale, sofern eine solche alternativ zur Verfügung gestellt werden soll.

Bezüglich bevorzugter Bereiche der Tiefe und Winkelerstreckung sowohl der Aussparungen als auch der dazwischen liegenden Segmente wird auf die Ansprüche 5 und 6 bzw. 15 und 16 verwiesen.

Bezüglich eines bevorzugten Bereiches für eine Überhöhung des wenigstens einen Segments wird auf die Ansprüche 8 bzw. 18 hingewiesen.

Die dem Knochen zugewandte Außenfläche ist vorzugsweise makrostrukturiert, insbesondere mit einer makrostrukturierten Schicht versehen. Damit wird die Verbindung zwischen der Schale bzw. Gelenkpfanne und dem natürlichen Knochen gefördert.

Bezüglich der Definition einer Flachprofilschale einerseits und Vollprofilschale andererseits wird auf Anspruch 10 verwiesen, wonach sich der Meridian einer Vollprofilschale über 180° erstreckt, während der Meridian einer Flachprofilschale sich über weniger als 180°, z.B. nur 145° oder 150° erstreckt.

Eine vorteilhafte Ausführungsform ist noch dadurch gekennzeichnet, daß die Wandstärke der Außenschale und/oder des Inlays oder bei einstückiger Ausführung der Schale die Wandstärke dieser ausgehend vom Pol der artikulierenden Fläche in Richtung nach radial außen bzw. in Richtung zur Äquatorialfläche hin stufenweise oder kontinuierlich zunimmt. Wesentlich ist also bei dieser Ausführungsform, daß der Abstand zwischen der artikulierenden Fläche und der Außenfläche jeweils am Pol kleiner ist als derjenige in der Äquatorialebene.

Es sei an dieser Stelle nochmals darauf hingewiesen, daß die erfindungsgemäßen Maßnahmen gleicherweise für eine modulare Schale einerseits und einstückige bzw. Monoblock-Schale andererseits gelten.

Nachstehen werden bevorzugte Ausführungsformen einer erfindungsgemäß ausgebildeten Hüftgelenkprothese bzw. einer dazugehörigen Gelenkpfanne anhand der beigefügten Zeichnungen näher beschrieben. Diese zeigen in:
- Fig. 1a, 1b und 1c: eine erste Ausführungsform einer erfindungsgemäß ausgebildeten Außenschale für eine Hüftgelenkprothese in unterschiedlichen perspektivischen Ansichten;
- Fig. 2a: eine vereinfachte Seitenansicht einer Hüftgelenkpfanne bzw. -schale zur Darstellung der Äquatorialebene;
- Fig. 2b: eine weitere Ausführungsform einer erfindungsgemäß ausgebildeten Hüftgelenkpfanne bzw. -schale, insbesondere Außenschale in Seitenansicht unter Darstellung von drei unterschiedlichen Aussparungen und Segmenten dazwischen;
- Fig. 2c: die Schale gemäß Fig. 2b in Draufsicht;
- Fig. 3: eine gegenüber der Schale gemäß den Fig. 2b, 2c abgewandelte Schale mit einem überhöhten Segment zwischen zwei benachbarten Aussparungen, in Seitenansicht;
- Fig. 4: ein Inlay für eine modulare Hüftgelenkpfanne bzw. -schale in Draufsicht; und
- Fig. 5: eine weitere abgewandelte Hüftgelenkpfanne bzw. -schale oder eines dazugehörigen Inlays im Längsschnitt.

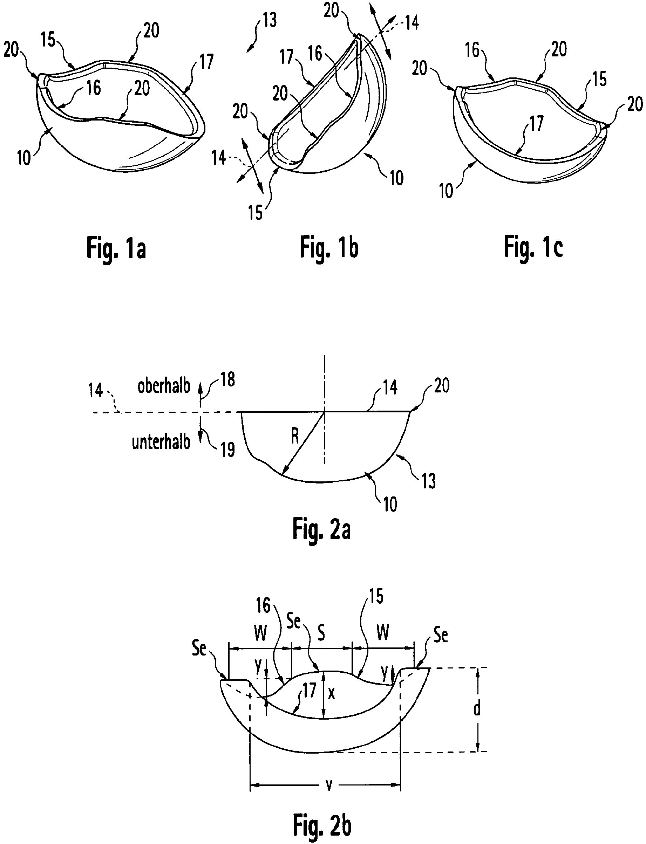

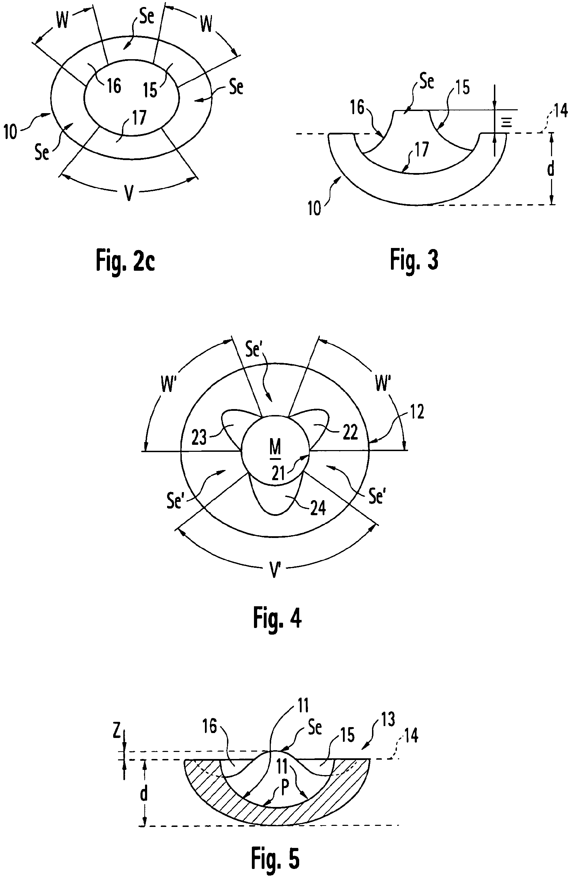

In den Figuren 1a, 1b und 1c ist eine erste Ausführungsform einer erfindungsgemäß ausgebildeten Hüftgelenkprothese dargestellt, bestehend aus einer metallenen Außenschale 10 und einem in der Außenschale 10 verankerbaren, hier nicht gezeigten Inlay, welches eine artikulierende bzw. Lagerfläche für einen femuralen Gelenkkopf umfaßt. Ein dazugehöriges Inlay bzw. eine bevorzugte Ausführungsform eines solchen Inlays ist in Fig. 4 dargestellt und mit der Bezugsziffer 12 gekennzeichnet. Die artikulierende bzw. Lagerfläche für einen femuralen Gelenkkopf weist die Bezugsziffer 11 auf. Die übergeordnete Bezugsziffer für die modulare oder einstückige Hüftgelenkprothese bzw. -pfanne ist die Bezugsziffer 13.

Entsprechend den Figuren 1a, 1b, 1c weist die Außenschale 10 drei sich jeweils unter ihre bzw. unterhalb ihrer Äquatorialebene 14 (siehe dazu auch Fig. 2a) erstreckende Aussparungen 15, 16 und 17 auf, in die ein Schenkelhals kollisionsfrei hineinbewegbar ist.

In Fig. 2a ist die Äquatorialebene 14 schematisch dargestellt. Der Pfeil 18 weist in Richtung oberhalb und der Pfeil 19 in Richtung unterhalb der Äquatorialebene 14. Im übrigen zeigt Fig. 2a nur schematisch eine Hüftgelenkprothese 13 in Seitenansicht. Fig. 2a dient im wesentlichen nur der Erläuterung der Äquatorialebene und des Bereichs oberhalb und unterhalb derselben. Des weiteren stellt Fig. 2a schematisch eine 180°-Pfanne dar. D.h. der Meridian mit dem Radius "R" erstreckt sich über 180°.

Der Rand 20 der Außenschale 10 erstreckt sich zwischen den Aussparungen 15, 16 und 17 entweder in, unter oder über der Äquatorialebene 14; diesbezüglich wird noch Näheres ausgeführt anhand der Figuren 2b, 2c und 3.

Wie die Figuren 1a, 1b und 1c ebenso wie die Figuren 2b und 2c erkennen lassen, sind die drei Aussparungen 15, 16 und 17 etwa gleichmäßig über den Umfang verteilt angeordnet. Der Winkelabstand beträgt also etwa 120°. Eine der drei Vertiefungen, nämlich die Vertiefung 17, ist tiefer und länger ausgebildet als die beiden anderen Vertiefungen 15, 16.

Entsprechend Fig. 2b sind die Abmessungen der größten Aussparung 17 im Vergleich zu den beiden kleineren Aussparungen 15, 16 bei einer Gesamthöhe "d" der Außenschale 10 wie folgt:

| | | |
|---|---|---|
| a) | Tiefe (x) der größten Aussparung 17: | 0,02 d ≤ x ≤ 0,4 d |
| b) | Tiefe (y) der kleineren Aussparungen 15 und 16: | 0,02 d ≤ y ≤ 0,2 d |
| c) | (Winkel-)Erstreckung (v) der größten | |
| | Aussparung 17 über den Umfang: | 90° ≤ v ≤ 165° |
| d) | (Winkel-)Erstreckung (w) der kleineren | |
| | Aussparungen 15, 16 über den Umfang: | 40° ≤ w ≤ 70°. |

Die Segmente (Se) bzw. Randbereiche der Außenschale 10 zwischen den Aussparungen 15, 16, 17 erstrecken sich über einen Winkelbereich (s) von 5° ≤ s ≤ 270°.

Entsprechend Fig. 2c betragen die Winkelbereiche (w) für die Aussparungen 15, 16 jeweils etwa 45° bis 50°, während der Winkelbereich (v), über den sich die größte Aussparung 17 erstreckt, etwa 100° beträgt.

Bei der Ausführungsform gemäß Fig. 3 ist eines der Segmente (Se) zwischen den beiden Aussparungen 15 ,16 gegenüber der Äquatorialebene 14 überhöht, wobei die Überhöhung (Z) im Bereich von 0,02 d ≤ Z ≤ 0,2 d liegt.

Wie vorstehend dargelegt, können Tiefe und Länge der Aussparungen 15, 16, 17 unterschiedlich sein. Gleiches gilt auch für die Form der Aussparungen.

Auch können die Überhöhung und die Länge der zwischen den Aussparungen angeordneten Segmente bzw. Randbereiche unterschiedlich ausgebildet sein. Letztlich hängt dies von der bevorzugten Anpassung an eine vorgegebene Anatomie ab, und auch von der zu erzielenden Primärstabilität. Dies gilt insbesondere für sogenannte Flachprofilpfannen bzw. -schalen.

Das Inlay 12 kann entweder entsprechend der Außenschale 10 ausgespart sein. Alternativ ist es auch denkbar, nur das Inlay 12 auszusparen, wobei die Aussparung in beiden Varianten jeweils vom Umfangsrand 21 der artikulierenden Fläche 11 ausgeht. In Fig. 4 ist schematisch ein solches Inlay in Draufsicht dargestellt, wobei die vorgenannten Aussparungen im Inlay dort mit den Bezugsziffern 22, 23 und 24 gekennzeichnet sind. Die Aussparung 24 ist hinsichtlich ihrer Winkelerstreckung und Tiefe größer ausgebildet als die beiden übrigen Aussparungen 22, 23. Insofern deckt sich die Ausbildung der Aussparungen 22, 23 und 24 mit den vorbeschriebenen Aussparungen 15, 16 und 17 der Außenschale 10.

Bezüglich der Abmessungen der Aussparungen 22, 23 und 24 im Inlay bei einer Gesamthöhe (d') des Inlays 12 sei erwähnt, daß die Abmessungen der größten Aussparung 24 im Vergleich zu den beiden kleineren Aussparungen 22, 23 wie folgt sind:

| | | |
|---|---|---|
| a) | Tiefe (x') der größten Aussparung 24: | 0,02 d' ≤ x' ≤ 0,4 d' |
| b) | Tiefe (y') der kleineren Aussparungen 22, 23: | 0,02 d' ≤ y' ≤ 0,2 d' |
| c) | (Winkel-)Erstreckung (v') der größten | |
| | Aussparung 24 über den Umfang: | 90° ≤ v' ≤ 165° |
| d) | (Winkel-)Erstreckung (w') der kleineren | |
| | Aussparungen 22, 23 über den Umfang: | 40° ≤ w' ≤ 70°. |

Auch beim Inlay erstrecken sich die Segmente (Se') bzw. Randbereiche zwischen den Aussparungen 22, 23, 24 über einen Winkelbereich (s') von 5° ≤ s' ≤ 270°.

Auch kann wenigstens eines der Segmente (Se') gegenüber der Äquatorialebene überhöht sein, wobei auch in diesem Fall die Überhöhung (Z') im Bereich von 0,02 d' ≤ Z' ≤ 0,2 d' liegt.

Schließlich können auch die Aussparungen 22, 23, 24 nicht nur unterschiedlich lang und tief, sondern auch unterschiedlich geformt bzw. gewölbt sein. Gleiches gilt auch für die zwischen den Aussparungen angeordneten Segmente (Se').

Wie bereits oben ausgeführt, können Außenschale 10 und Inlay 12 modular, aber auch einstückig bzw. als Monoblock ausgebildet sein, so wie dies z.B. in Fig. 5 dargestellt ist. Dabei zeigt Fig. 5 noch ein weiteres vorteilhaftes Merkmal, welches bei Bedarf sämtliche Ausführungsformen aufweisen können. Dieses Merkmal ist dadurch gekennzeichnet, daß die Wandstärke der Schale 13 ausgehend vom Pol (P) der artikulierenden Fläche 11 in Richtung nach radial außen bzw. in Richtung zur Äquatorialfläche 14 hin kontinuierlich zunimmt. Auch eine stufenweise Zunahme der Wandstärke ist denkbar.

Bei modularem Aufbau kann sich diese Maßnahme auf die Außenschale 10 oder nur das Inlay 12 oder auf beide Bauteile beziehen.

Wie ebenfalls bereits einleitend ausgeführt, besteht das die artikulierende Fläche 11 umfassende Bauteil aus einem Polymer, insbesondere Polyethylen, Keramik oder Metall. Letztlich hängt dies von der gewünschten Paarung zwischen der artikulierenden Fläche einerseits und dem komplementären Gelenkkopf andererseits ab. Da es sich diesbezüglich um an sich bekannte Maßnahmen handelt, erübrigt sich eine weitere Ausführung dazu.

Gleiches gilt für die Makrostrukturierung der dem Knochen zugewandten Außenfläche der beschriebenen Hüftgelenkpfanne. Diese kann auch durch eine makrostrukturierte Beschichtung erhalten werden.

Sämtliche in den Anmeldungsunterlagen offenbarten Merkmale werden als erfindungswesentlich beansprucht, soweit sie einzeln oder in Kombination gegenüber dem Stand der Technik neu sind.

### Bezugszeichen

- 10: Außenschale
- 11: artikulierende Fläche
- 12: Inlay
- 13: Hüftgelenkprothese bzw. -pfanne
- 14: Äquatorialebene
- 15: Aussparung
- 16: Aussparung
- 17: Aussparung
- 18: Pfeil
- 19: Pfeil
- 20: Rand bzw. Segment
- 21: Umfangsrand
- 22: Aussparung (Inlay)
- 23: Aussparung (Inlay)
- 24: Aussparung (Inlay)

## Patentansprüche

1. Hüftgelenkprothese (13) bestehend aus einer metallenen Außenschale (10) und einem in der Außenschale (10) verankerbaren Inlay (12), welches eine artikulierende bzw. Lagerfläche (11) für einen femuralen Gelenkkopf umfaßt,
wobei die Außenschale (10) vorzugsweise wenigstens anterior eine sich unter ihre bzw. unterhalb ihrer Äquatorialebene (14) erstreckende Aussparung (15, 16, 17) aufweist, in die ein Schenkelhals kollisionsfrei hineinbewegbar ist,
wobei die Außenschale (10) drei etwa gleichmäßig über den Umfang verteilt angeordnete Aussparungen (15, 16, 17) aufweist, **dadurch gekennzeichnet, daß** eine davon tiefer
und länger ist als die beiden anderen.

2. Hüftgelenkprothese nach Anspruch 1,
**dadurch gekennzeichnet, daß**
der Rand (20) der Außenschale (10) zwischen der wenigstens einen Aussparung (15, 16, 17) sich in, unter oder über der Äquatorialebene (14) erstreckt.

3. Hüftgelenkprothese nach Anspruch 1 oder 2,
**dadurch gekennzeichnet, daß**
die Außenschale (10) wenigstens zwei Aussparungen (15, 16, 17) aufweist, derart, daß sie für eine Implantation sowohl links als auch rechts geeignet ist.

4. Hüftgelenkprothese nach einem der Ansprüche 1 bis 3,
**dadurch gekennzeichnet, daß**
bei einer Gesamthöhe (d) der Außenschale (10) die Abmessungen der größten Aussparung (17) im Vergleich zu den beiden kleineren Aussparungen (15, 16) wie folgt sind:
| | | |
|---|---|---|
| a) | Tiefe (x) der größten Aussparung (17): | 0,02 d ≤ x ≤ 0,4 d |
| b) | Tiefe (y) der kleineren Aussparungen (15, 16): | 0,02 d ≤ y ≤ 0,2 d |
| c) | (Winkel-)Erstreckung (v) der größten | |
| | Aussparung (17) über den Umfang: | 90° ≤ v ≤ 165° |
| d) | (Winkel-)Erstreckung (w) der kleineren | |
| | Aussparungen (15, 16) über den Umfang: | 40° ≤ w ≤ 70°. |

5. Hüftgelenkprothese nach einem der Ansprüche 1 bis 4,
**dadurch gekennzeichnet, daß**
die Segmente (Se) bzw. Randbereiche der Außenschale (10) zwischen der bzw. den Aussparungen (15, 16, 17) sich über einen Winkelbereich (s) von 5° ≤ s ≤ 270° erstrecken.

6. Hüftgelenkprothese nach einem der Ansprüche 1 bis 5,
**dadurch gekennzeichnet, daß**
wenigstens ein Segment (Se) bzw. Randbereich zwischen der wenigstens einen Aussparung (15, 16; 17) gegenüber der Äquatorialebene (14) überhöht ist.

7. Hüftgelenkprothese nach Anspruch 6,
**dadurch gekennzeichnet, daß**
die Überhöhung (Z) des wenigstens einen Segments im Bereich von 0,02 d ≤ Z ≤ 0,2 d liegt.

8. Hüftgelenkprothese nach einem der Ansprüche 1 bis 7,
**dadurch gekennzeichnet, daß**
die Tiefe, Länge und auch Form der Aussparungen (15, 16, 17) unterschiedlich sind, ebenso wie die Überhöhung und Länge der dazwischen angeordneten Segmente bzw. Randbereiche der Außenschale (10).

9. Hüftgelenkprothese nach einem der Ansprüche 1 bis 8,
**dadurch gekennzeichnet, daß**
die Außenschale als Vollprofilschale (Meridian über 180° erstreckend), oder als Flachprofilschale (Meridian über weniger als 180°) ausgebildet ist.

10. Hüftgelenkprothese, insbesondere nach einem der Ansprüche 1 bis 9,
**dadurch gekennzeichnet, daß**
das Inlay (12) entweder entsprechend der Außenschale (10) ausgespart, oder daß nur das Inlay (12), und zwar ausgehend vom Umfangsrand (21) der artikulierenden Fläche (11) ausgespart ist.

11. Hüftgelenkprothese nach Anspruch 10,
**dadurch gekennzeichnet, daß**
der Umfangsrand bzw. das Segment (Se') des Inlays (12) zwischen der wenigstens einen Aussparung (22, 23, 24) sich in, unter oder über der Äquatorialebene (14) erstreckt.

12. Hüftgelenkprothese nach Anspruch 10 oder 11,
**dadurch gekennzeichnet, daß**
das Inlay (12) wenigstens zwei Aussparungen (22, 23, 24) aufweist, derart, daß es für eine Implantation sowohl links als auch rechts geeignet ist.

13. Hüftgelenkprothese nach einem der Ansprüche 10 bis 12,
**dadurch gekennzeichnet, daß**
das Inlay (12) drei etwa gleichmäßig über den Umfang verteilt angeordnete Aussparungen (22, 23, 24) aufweist, wobei eine (24) davon tiefer und länger sowohl in Umfangs- als auch Radialrichtung ausgebildet ist als die beiden anderen.

14. Hüftgelenkprothese nach Anspruch 13,
**dadurch gekennzeichnet, daß**
bei einer Gesamthöhe (d') des Inlays (12) die Abmessungen der größten Aussparung (24) im Vergleich zu den beiden kleineren Aussparungen (22, 23) wie folgt sind:
| | | |
|---|---|---|
| a) | Tiefe (x') der größten Aussparung (24): | 0,02 d' ≤ x' ≤ 0,4 d' |
| b) | Tiefe (y') der kleineren Aussparungen (22, 23): | 0,02 d' ≤ y' ≤ 0,2 d' |
| c) | (Winkel-)Erstreckung (v') der größten | |
| | Aussparung (24) über den Umfang: | 90° ≤ v' ≤ 165° |
| d) | (Winkel-)Erstreckung (w') der kleineren | |
| | Aussparungen (22, 23) über den Umfang: | 40° ≤ w' ≤ 70°. |

15. Hüftgelenkprothese nach Anspruch 13 und/oder 14,
**dadurch gekennzeichnet, daß**
die Segmente (Se') bzw. Randbereiche des Inlays (12) zwischen der bzw. den Aussparungen (22, 23, 24) sich über einen Winkelbereich (s') von 5° ≤ s' ≤ 270° erstrecken.

16. Hüftgelenkprothese nach einem der Ansprüche 13 bis 15,
**dadurch gekennzeichnet, daß**
wenigstens ein Segment (Se') bzw. Randbereich zwischen der wenigstens einen Aussparung (22, 23, 24) gegenüber der Äquatorialebene (14) überhöht ist.

17. Hüftgelenkprothese nach Anspruch 16,
**dadurch gekennzeichnet, daß**
die Überhöhung (Z') des wenigstens einen Segments im Bereich von 0,02 d' ≤ Z' ≤ 0,2 d'
liegt.

18. Hüftgelenkprothese nach einem der Ansprüche 13 bis 17,
**dadurch gekennzeichnet, daß**
die Tiefe, Länge und auch Form der Aussparungen (22, 23, 24) unterschiedlich sind, ebenso wie die Überhöhung und Länge der dazwischen angeordneten Segmente (Se') bzw. Randbereiche des Inlays (12).

19. Hüftgelenkprothese nach einem der Ansprüche 1 bis 18,
**dadurch gekennzeichnet, daß**
Außenschale (10) und Inlay (12) einstückig oder modular ausgebildet sind.

20. Hüftgelenkprothese nach einem der Ansprüche 1 bis 19,
**dadurch gekennzeichnet, daß**
das die artikulierende Fläche (11) umfassende Bauteil aus einem Polymer, insbesondere Polyethylen, Keramik oder Metall besteht.

21. Hüftgelenkprothese nach einem der Ansprüche 1 bis 20,
**dadurch gekennzeichnet, daß**
die dem Knochen zugewandte Außenfläche makrostrukturiert, insbesondere mit einer makrostrukturierten Schicht versehen ist.

22. Hüftgelenkprothese nach einem der Ansprüche 1 bis 21,
**dadurch gekennzeichnet, daß**
die Wandstärke der Außenschale (10), und/oder des Inlays (12), oder bei einstückiger Ausbildung der Schale (13) die Wandstärke derselben ausgehend vom Pol (P) der artikulierenden Fläche (11) in Richtung nach radial außen bzw. in Richtung zur Äquatorialfläche (14) hin stufenweise oder kontinuierlich zunimmt.

## Claims

1. Hip joint prosthesis (13) consisting of a metal outer shell (10) and an inlay (12) fixable in said outer shell (10) which comprises an articulating or bearing surface (11) for a femoral articular head,
wherein the outer shell (10) preferably has at least anteriorly a recess (15, 16, 17) extending under or underneath its equatorial plane (14) into which a femoral neck can be eased collision-free,
wherein
the outer shell (10) has three recesses (15, 16, 17) spaced approximately evenly around the periphery, **characterised in that** one of them is deeper and longer than the other two.

2. Hip joint prosthesis according to claim 1,
**characterised in that**
the edge (20) of the outer shell (10) extends between the at least one recess (15, 16, 17) in, under or over the equatorial plane (14).

3. Hip joint prosthesis according to claim 1 or 2,
**characterised in that**
the outer shell (10) has at least two recesses (15, 16, 17) such that it is suitable for implantation both on the left and also on the right.

4. Hip joint prosthesis according to one of claims 1 to 3,
**characterised in that**
with an overall height (d) of the outer shell (10), the dimensions of the largest recess (17) compared to the two smaller recesses (15, 16) are as follows:
| | | |
|---|---|---|
| a) | Depth (x) of the largest recess (17): | 0.02 d ≤ x ≤ 0.4 d |
| b) | Depth (y) of the smaller recesses (15, 16): | 0.02 d ≤ y ≤ 0.2 d |
| c) | (Angle) extension (v) of the largest recess (17) over the periphery: | 90° ≤ v ≤ 165° |
| d) | (Angle) extension (w) of the smaller recesses (15, 16) over the periphery: | 40° ≤ w ≤ 70° |

5. Hip joint prosthesis according to one of claims 1 to 4,
**characterised in that**
the segments (Se) or edge regions of the outer shell (10) between the recesses (15, 16, 17) extend over an angle range (s) of 5° ≤ s ≤ 270°.

6. Hip joint prosthesis according to one of claims 1 to 5,
**characterised in that**
at least one segment (Se) or edge region between the at least one recess (15, 16, 17) is superelevated with respect to the equatorial plane (14).

7. Hip joint prosthesis according to claim 6,
**characterised in that**
the superelevation (Z) of the at least one segment is in the region of 0.02 d ≤ Z ≤ 0.2 d.

8. Hip joint prosthesis according to one of claims 1 to 7,
**characterised in that**
the depth, length and also shape of the recesses (15, 16, 17) are different, as well as the superelevation and length of the segments or edge regions of the outer shell (10) arranged between them.

9. Hip joint prosthesis according to one of claims 1 to 8,
**characterised in that**
the outer shell is formed as a full profile shell (meridian extending over 180°), or as a flat profile shell (meridian over less than 180°).

10. Hip joint prosthesis, in particular according to one of claims 1 to 9,
**characterised in that**
the inlay (12) is recessed either corresponding to the outer shell (10), or that only the inlay (12) is recessed, that is proceeding from the peripheral edge (21) of the articulating surface (11).

11. Hip joint prosthesis according to claim 10,
**characterised in that**
the peripheral edge or the segment (Se') of the inlay (12) extends between the at least one recess (22, 23, 24) in, under or over the equatorial plane (14).

12. Hip joint prosthesis according to claim 10 or 11,
**characterised in that**
the inlay (12) has at least two recesses (22, 23, 24) such that it is suitable for implantation both on the left and also on the right.

13. Hip joint prosthesis according to one of claims 10 to 12,
**characterised in that**
the inlay (12) has three recesses (22, 23, 24) spaced approximately evenly around the periphery, wherein one (24) of them is formed deeper and longer both in the peripheral and also the radial direction than the other two.

14. Hip joint prosthesis according to claim 13,
**characterised in that**
with an overall height (d') of the inlay (12), the dimensions of the largest recess (24) compared to the two smaller recesses (22, 23) are as follows:
| | |
|---|---|
| a) Depth (x') of the largest recess (24): | 0.02 d' ≤ x' ≤ 0.4 d' |
| b) Depth (y') of the smaller recesses (22, 23): | 0.02 d' ≤ y' ≤ 0.2 d' |
| c) (Angle) extension (v') of the largest recess (24) over the periphery: | 90° ≤ v' ≤ 165° |
| d) (Angle) extension (w') of the smaller recesses (22, 23) over the periphery: | 40° ≤ w' ≤ 70°. |

15. Hip joint prosthesis according to claim 13 and/or 14,
**characterised in that**
the segments (Se') or edge regions of the inlay (12) between the recesses (22, 23, 24) extend over an angle range (s') of 5° ≤ s' ≤ 270°.

16. Hip joint prosthesis according to one of claims 13 to 15,
**characterised in that**
at least one segment (Se') or edge region between the at least one recess (22, 23, 24) is superelevated with respect to the equatorial plane (14).

17. Hip joint prosthesis according to claim 16,
**characterised in that**
the superelevation (Z') of the at least one segment is in the region of 0.02 d' ≤ Z' ≤ 0.2 d'.

18. Hip joint prosthesis according to one of claims 13 to 17,
**characterised in that**
the depth, length and also shape of the recesses (22, 23, 24) are different, as well as the superelevation and length of the segments (Se') or edge regions of the inlay (12) arranged between them.

19. Hip joint prosthesis according to one of claims 1 to 18,
**characterised in that**
outer shell (10) and inlay (12) are formed in one piece or are modular.

20. Hip joint prosthesis according to one of claims 1 to 19,
**characterised in that**
the component encompassing the articulating surface (11) is comprised of a polymer, in particular polyethylene, ceramic or metal.

21. Hip joint prosthesis according to one of claims 1 to 20,
**characterised in that**
the outer surface directed towards the bone is macro-structured, in particular it is provided with a macro-structured layer.

22. Hip joint prosthesis according to one of claims 1 to 21,
**characterised in that**
the wall thickness of the outer shell (10), and/or the inlay (12), or, with a one-piece configuration of the shell (13), the wall thickness thereof, proceeding from the pole (P) of the articulating surface (11), increases incrementally or continuously radially outwards or towards the equatorial plane (14).

## Revendications

1. Prothèse articulaire de la hanche (13) constituée d'une coque extérieure métallique (10) et d'un insert (12) pouvant être ancré dans la coque extérieure (10), qui comprend une surface d'articulation ou d'appui (11) pour une tête d'articulation fémorale,
la coque extérieure (10) comprenant de préférence au moins en position antérieure un évidement (15, 16, 17), s'étendant sous son plan équatorial (14) ou en-dessous de ce dernier, évidement dans lequel un col de fémur peut se déplacer dans un sens et dans l'autre sans collision,
la coque extérieure (10) comprenant trois évidements (15, 16, 17), disposée en étant répartis d'une manière approximativement équidistante sur la périphérie,
**caractérisée en ce que** l'un de ceux-ci est plus profond et plus long que les deux autres.

2. Prothèse articulaire de la hanche selon la revendication 1, **caractérisée en ce que** le bord (20) de la coque extérieure (10) s'étend entre le ou les évidements (15, 16, 17), dans, en-dessous ou au-dessus du plan équatorial (14).

3. Prothèse articulaire de la hanche selon la revendication 1 ou 2, **caractérisée en ce que** la coque extérieure (10) comprend au moins deux évidements (15, 16, 17) de telle sorte qu'une implantation puisse se faire tant à gauche qu'à droite.

4. Prothèse articulaire de la hanche selon l'une des revendications 1 à 3, **caractérisée en ce que**, pour une hauteur totale (d) de la coque extérieure (10), les dimensions de l'évidement le plus grand (17), par comparaison avec les deux autres évidements plus petits (15, 16), sont les suivantes :
a) profondeur (x) de l'évidement le plus grand (17) : 0,02 d ≤ x ≤ 0,4 d
b) profondeur (y) des évidements plus petits (15, 16) : 0,02 d ≤ y ≤ 0,2 d
c) extension (angulaire) (v) de l'évidement le plus grand (17) sur la périphérie : 90° ≤ v ≤ 165°
d) extension (angulaire) (w) des évidements plus petits (15,16) sur la périphérie : 40° ≤ w ≤ 70°.

5. Prothèse articulaire de la hanche selon l'une des revendications 1 à 4, **caractérisée en ce que** les segments (Se) ou les zones de bordure de la coque extérieure (10) s'étendent, entre le ou les évidements (15, 16, 17), sur une plage angulaire (s) de 5° ≤ s ≤ 270°.

6. Prothèse articulaire de la hanche selon l'une des revendications 1 à 5, **caractérisée en ce qu'**au moins un segment (Se) ou au moins une zone de bordure est, entre le ou les évidements (15, 16, 17), surélevé par rapport au plan équatorial (14).

7. Prothèse articulaire de la hanche selon la revendication 6, **caractérisée en ce que** la surélévation (Z) du ou des segments est comprise dans la plage de 0,02 d ≤ Z ≤ 0,2 d.

8. Prothèse articulaire de la hanche selon l'une des revendications 1 à 7, **caractérisée en ce que** la profondeur, la longueur et aussi la forme des évidements (15, 16, 17) sont différentes, tout comme la surélévation et la longueur des segments ou zones de bordure, disposées entre eux, de la coque extérieure (10).

9. Prothèse articulaire de la hanche selon l'une des revendications 1 à 8, **caractérisée en ce que** la coque extérieure est configurée comme une coque en profilé plein (méridien s'étendant sur 180°) ou comme une coque en profilé plat (méridien sur moins de 180°).

10. Prothèse articulaire de la hanche, en particulier selon l'une des revendications 1 à 9, **caractérisée en ce que** l'insert (12) ou bien est évidé en fonction de la coque extérieure (10), ou bien l'insert (12) est évidé seulement en partant plus précisément du bord périphérique (21) de la surface d'articulation (11).

11. Prothèse articulaire de la hanche selon la revendication 10, **caractérisée en ce que** le bord périphérique ou le segment (Se') de l'insert (12) s'étend entre le ou les évidements (22, 23, 24), dans, en-dessous ou au-dessus du plan équatorial (14).

12. Prothèse articulaire de la hanche selon la revendication 10 ou 11, **caractérisée en ce que** l'insert (12) comprend au moins deux évidements (22, 23, 24), de telle sorte qu'ils conviennent à une implantation tant à gauche qu'à droite.

13. Prothèse articulaire de la hanche selon l'une des revendications 10 à 12, **caractérisée en ce que** l'insert 12 comprend trois évidements (22, 23, 24), disposés en étant répartis d'une manière approximativement régulière sur la périphérie, l'un (24) de ceux-ci étant configuré plus profond et plus long, tant dans la direction périphérique que dans la direction radiale, que les deux autres.

14. Prothèse articulaire de la hanche selon la revendication 13, **caractérisée en ce que**, pour une hauteur totale (d') de l'insert (12), les dimensions de l'évidement le plus grand (24), par comparaison avec les deux évidements plus petits (22, 23), sont les suivantes :
a) profondeur (x') de l'évidement le plus (24) : 0,02 d' ≤ x' ≤ 0,4 d'
b) profondeur (y') des évidements plus petits (22, 23) : 0,02 d' ≤ y' ≤ 0,2 d'
c) extension (angulaire) (v') de l'évidement le plus grand (24) sur la périphérie : 90° ≤ v' ≤ 165°
d) extension (angulaire) (w') des évidements plus petits (22, 23) sur la périphérie : 40° ≤ w' ≤ 70°.

15. Prothèse articulaire de la hanche selon la revendication 13 et/ou 14, **caractérisée en ce que** les segments (Se') ou les zones de bordure de l'insert (12) s'étendent entre le ou les évidements (22, 23, 24) sur une plage angulaire (s') de 5° ≤ s' ≤ 270°.

16. Prothèse articulaire de la hanche selon l'une des revendications 13 à 15, **caractérisée en ce qu'**au moins un segment (Se') ou au moins une zone de bordure entre le ou les évidements (22, 23, 24) est surélevé par rapport au plan équatorial (14).

17. Prothèse articulaire de la hanche selon la revendication 16, **caractérisée en ce que** la surélévation (Z') du ou des segments est comprise dans la plage de 0,02 d' ≤ Z' ≤ 0,2 d'.

18. Prothèse articulaire de la hanche selon l'une des revendications 13 à 17, **caractérisée en ce que** la profondeur, la longueur et aussi la forme des évidements (22, 23, 24) sont différentes, tout comme la surélévation et la longueur des segments (Se') ou zones de bordure, disposés entre eux, de l'insert (12).

19. Prothèse articulaire de la hanche selon l'une des revendications 1 à 18, **caractérisée en ce que** la coque extérieure (10) et l'insert (12) sont configurés en une seule pièce ou d'une manière modulaire.

20. Prothèse articulaire de la hanche selon l'une des revendications 1 à 19, **caractérisée en ce que** le composant comprenant la surface d'articulation (11) est constitué d'un polymère, en particulier de polyéthylène, d'une céramique ou d'un métal.

21. Prothèse articulaire de la hanche selon l'une des revendications 1 à 20, **caractérisée en ce que** la surface extérieure en regard de l'os est macrostructurée, et en particulier est pourvue d'une couche macrostructurée.

22. Prothèse articulaire de la hanche selon l'une des revendications 1 à 21, **caractérisée en ce que** l'épaisseur de paroi de la coque extérieure (10) et/ou de l'insert (12), ou encore, dans le cas d'une configuration en une seule pièce de la coque (13), son épaisseur de paroi, augmente par paliers ou d'une manière continue, en partant du pôle (P) de la surface d'articulation (11) dans la direction allant radialement vers l'extérieur ou dans la direction allant vers la surface équatoriale (14).
